# EUROPEAN PATENT SPECIFICATION

(11) **EP 3 852 818 B1**
(45) Date of publication and mention of the grant of the patent: **13.12.2023**
(21) Application number: 18871805.0
(22) Date of filing: 18.09.2018
(51) Int. Cl.: A61L 2/12, A61F 5/455

(54) **CONTAINER FOR CLEANING AND STERILIZING MENSTRUAL CUPS**
BEHÄLTER ZUM REINIGEN UND STERILISIEREN VON MENSTRUATIONSBECHERN
RÉCIPIENT POUR LE NETTOYAGE ET LA STÉRILISATION DE COUPES MENSTRUELLES

(43) Date of publication of application: 28.07.2021
(73) Proprietor: SHE IS LUCID SPOLKA Z OGRANICZONA ODPOWIEDZIALNOSCIA, 02-531 Warszawa (PL)
(72) Inventor: LEQUAY, Paul, 38260 Bossieu (FR); SLOWIK, Iga, 40-031 Katowice (PL)
(74) Representative: AOMB Polska Sp. z.o.o.
(86) International application number: PCT/PL2018/050048
(87) International publication number: WO 2020/060424

(56) References cited:
- WO-A1-91/12028
- WO-A1-2011/110666
- WO-A1-2014/015975
- WO-A1-2018/138963
- KR-B1- 101 881 521
- US-A- 4 877 624
- US-A1- 2003 217 423

## Description

### Technical Field

The present invention relates to the field of a feminine hygiene and more specifically to a portable container for cleaning and sterilizing menstrual cups. Such a container is composed of materials suitable for use with microwaves but also for medical contact in menstrual hygiene management.

### Background Art

Menstrual cups are well-known in the art as eco-friendly and reusable solution to manage menstruation instead of pads or tampons. Typically, a menstrual cup is made of medical-grade silicone and contains a bell-shaped portion with a tapered bottom for collecting menses and gripping means at the bottom of this portion, such as a stem, for insertion and removal during menstrual cycles.

Known menstrual cups and microwaveable containers for sterilisation of objects are disclosed for example in documents US4877624A, WO2011110666A1, WO9112028A1, EP3323392A1, WO2014056258A1, WO2011013954A2.

Menstrual cups are easy to use for long-term but needs to be emptied every 8-12 hours and then cleaned before reusing. During these operations, it is important to safely manipulate the menstrual cup and thoroughly remove the blood or any materials collected in the menstrual cup.

Current techniques require use of disposable wet wipes or to hand-wash the menstrual cup with water in a sink or above a toilet. This may either produce waste or spill blood drops around the sink or the toilet contaminating both hands and the environment. In addition to that, if not done at home, women may experience psychological pressure and physical impediments such as the lack of privacy or lack of access to clean water or clean cloth or toilet paper to dry the menstrual cup. It can further cause a high risk of dropping the menstrual cup on a floor while handling it or a risk of contamination due to potentially unclean environment.

Once the menstrual cycle has ended and before another cycle begins, the menstrual cup must be sterilized by being submerged under water and exposed on microwave radiation, for instance by placing in a microwave oven for limited period of time. In order to do so, the menstrual cup is placed in a closed receptacle filled with water. One problem with this method is that the menstrual cup has to be manually operated with hands, that is to be rinsed with water, placed inside the receptacle and removed after sterilization. This has further drawbacks that water may easily spill out, closing the receptacle can be difficult, menstrual cup freely floats in the water and is not fully emerged.

Nevertheless, no device enables women to safely clean and sterilize a menstrual cup at the same time.

### Disclosure of Invention

Therefore, it is the object of the present invention to provide a portable and reusable cleaning and sterilizing container which eliminates the above-mentioned drawbacks, that is to enable safe process of cleaning and sterilizing menstrual cups, wherein contact with user hands is limited to minimum.

In accordance with the present invention there is provided a container for cleaning and sterilizing a menstrual cup, comprising a vessel with an open-top having a bottom, side walls defining an inner cavity for holding a cleaning liquid and for receiving the menstrual cup made of elastic material having a bell-shaped portion for collecting menses and gripping means, preferably in the form of a stem, for insertion and removal during menstrual cycle, a lid closing the open-top, the vessel and the lid being made of materials suitable for exposure to microwave radiation, wherein the lid comprises holding means for connecting the menstrual cup to the lid with the gripping means, the lid can be fitted on the open-top in a first mounting position with the holding means directed towards the inner cavity such that, when attached to the lid, the menstrual cup projects at least partially into the inner cavity.

The lid can be also mounted in a second mounting position when the lid is reversed with the holding means directed away from the inner cavity such that, when attached to the lid, the menstrual cup projects outwardly.

A container may be made of elastic material such as silicone or polymer or co-polymer plastic materials or combination thereof.

The holding means can have the form of projections pinching the gripping means and retaining the menstrual cup firmly in place.

Preferably, a central ring is provided between the vessel and the lid, the central ring encloses an upper part of the vessel and together with the lid forms a tight connection.

Preferably, a clamp ring with a portion at least partially enclosing the holding means is attached to the lid by screwing such that as the clamp ring screws on the lid said portion tightens up the gripping means and retains the menstrual cup firmly in place.

Advantageously, the container comprises a brush arranged in the inner cavity of the vessel, wherein the brush extends into the bell-shaped portion and is in contact or at close proximity with said portion when the lid is in the first mounting position with the menstrual cup attached to it.

The vessel is further advantageously provided with discharging means allowing the cleaning liquid and menses to be expelled after cleaning process and allowing a steam to escape during sterilization. The discharging means comprises a bottleneck arranged on the outside of the vessel, a spout, and a cap with a rod that enters the spout and closes it tightly. The bottleneck is provided with a recess that serves to pry and unlock the cap. On the outside of the vessel, close to the bottle neck, a protruding stripe with a pinch is provided, the pinch enters an opening in a portion of the cap and presses the cap against the vessel thereby securing the cap fitted in the spout.

In one embodiment the lid has a protruding portion on the side opposite to the side with the holding means, the protruding portion enables the lid to be retained in the second mounting position. The protruding portion has on its circumference bumps and recesses that serve the function of releasing the air from the vessel and ensure against an air tight seal.

Preferably, a flexible string is provided for secure and easy manipulation with user hands.

### Brief Description of Drawings

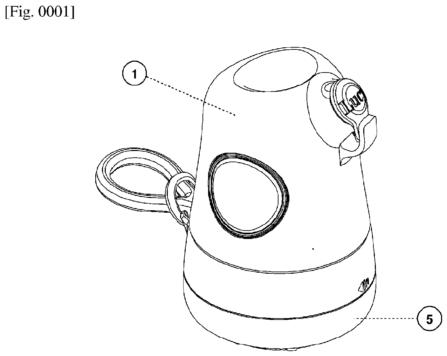

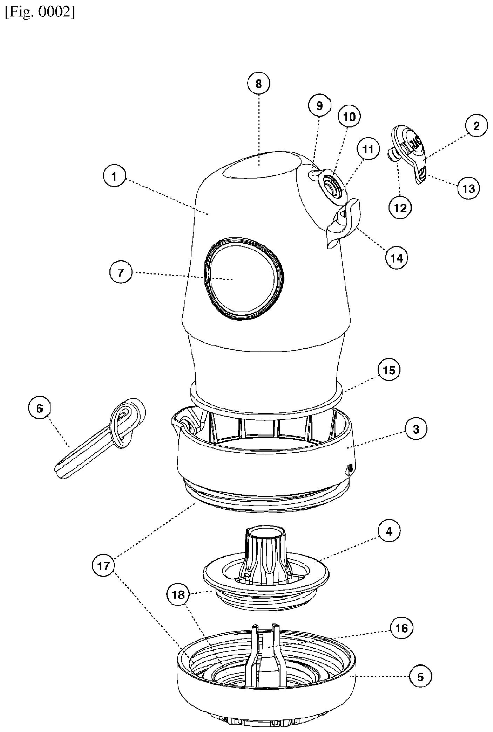

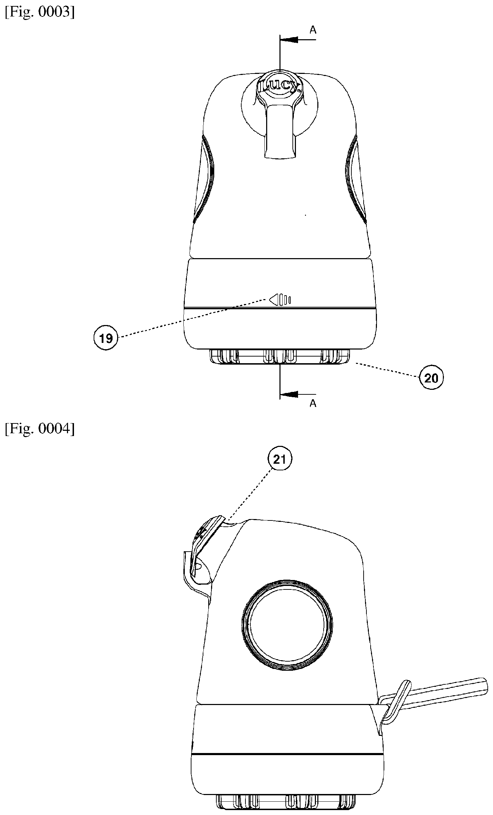

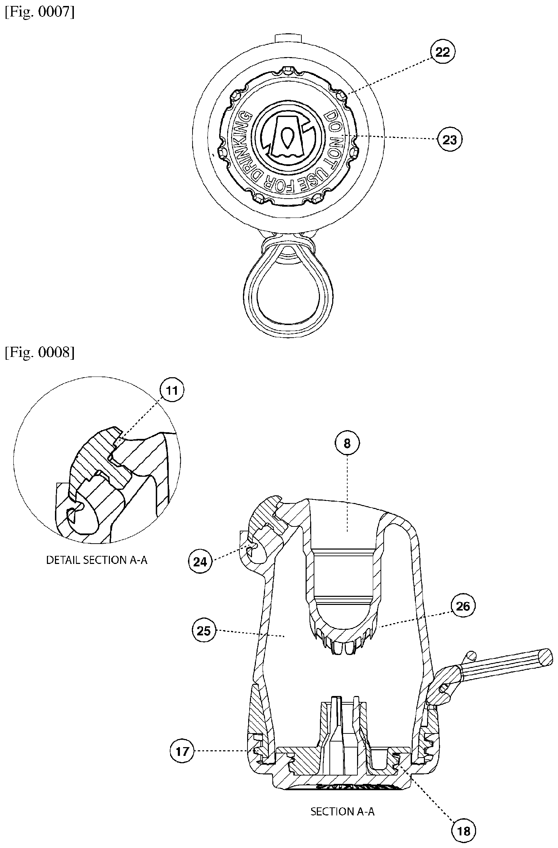

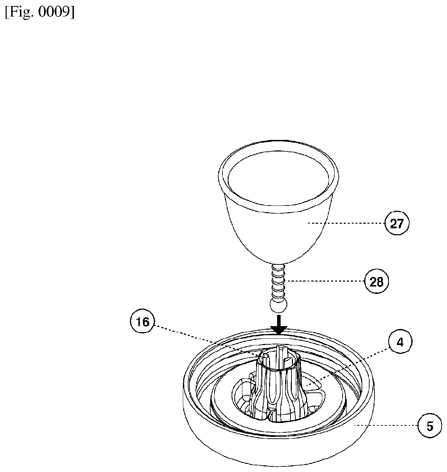

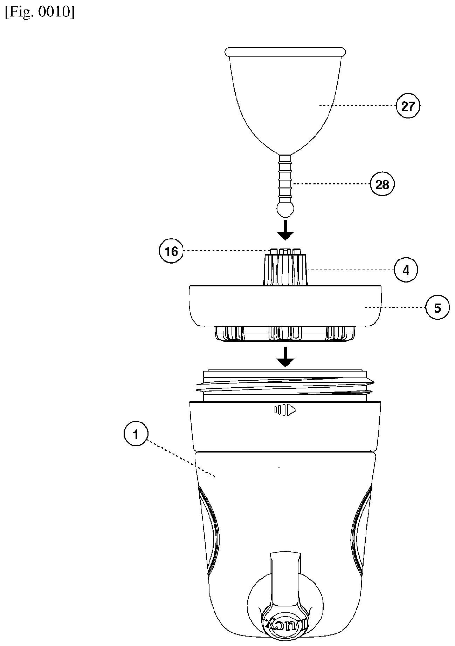

The invention will now be described by a way of example only with reference to the accompanying drawings, in which: Figure 1 is an isometric view of a container in accordance with the preferred embodiment of the present invention; Figure 2 is an exploded view of the container illustrated in Figure 1; Figures 3 and 4 and 5 are respectively front, side and rear views of the container illustrated in Figure 1; Figures 6 and 7 are respectively top and bottom projected views from Figure 1; Figure 8 is a section taken along line A-A of Figure 3; Figure 9 shows a process of attaching the menstrual cup to the lid; Figure 10 illustrates how components interact with each other with the lid being mounted in the second mounting position.

### Detailed description of preferred embodiments

Fig. 1 illustrates a container for cleaning and sterilizing menstrual cups in an isometric view.

A container is suitable for cleaning and sterilizing products of feminine hygiene such as a menstrual cup shown in Fig. 9 and 10 which is made of elastic material and has a bell-shaped portion 27 for collecting menses and gripping means 28 at a tapered bottom of the bell-shaped portion 27, preferably in the form of a stem, for insertion and removal during menstrual cycle.

A container in accordance with the preferred embodiment of the present invention comprises a vessel 1 with an open-top closed tightly with a lid 5. The vessel 1 has a bottom 8 and side walls defining an inner cavity 25 for holding a cleaning liquid and for receiving a menstrual cup.

The container is made of elastic materials suitable for medical contact and being subjected to heat in a microwave oven such as silicone, polymer or co-polymer plastics materials. It is to be understood that all the container can be made of elastic and flexible materials or the container is partly elastic and partly rigid.

Referring to Fig. 2 and Fig. 8 illustrating the container in exploded view and in cross-section respectively, the container may comprise a vessel 1, discharging means, a brush 26 arranged in a cavity 25, a flexible string 6, a central ring 3, a clamp ring 4, a lid 5 with holding means 16 and tactile and/or visible markers 7.

The lid 5 is reversible so that it can be fitted on the open-top in two mounting positions and further comprises holding means 16 for connecting the menstrual cup with the gripping means 28. The first mounting position is with the holding means 16 directed towards the inner cavity 25 such that, when attached to the lid 5, the menstrual cup projects at least partially into the inner cavity 25. The second mounting position is with the lid 5 reversed and the holding means 16 directed away from the inner cavity 25 such that the menstrual cup projects outwardly.

The holding means 16 can have the form of three projections. The stem 28 is pinched between said projections resulting in the menstrual cup being retained firmly in place.

The clamp ring 4 with a portion at least partially enclosing the holding means 16 is attached to the lid 5 by screwing such that as the clamp ring 4 screws on the lid 5 said portion tightens up the stem 28 and retains the menstrual cup firmly in place. The upper portion of the clamp ring 4 comprises grooves and recesses allowing the user to screw the clamp ring 4 on the lid 5 and to secure the clamp ring 4 on the holding means 16. On the outside of the walls of the lid 5 and on the inside of the bottom part of the clamp ring 4 mating threads 18 are provided enabling these two parts to connect each other.

On the inside of the lid 5 and on the outside of the central ring 3 there are provided mating threads 17 so as to connect and seal these two elements together. Compatible chamfers are located at the beginning of the each thread 17 to ease the positioning of said elements and to start screwing. A circular bulge arranged in the lid 5 creates a slot between the bulge and the thread 17 of the lid 5 where a rim 15 of the vessel 1 and the central ring 3 can slot in. It is meant to complete the sealing process of the vessel 1 while screwing the lid 5 to the central ring 3. The clamp ring 4 is connected by screwing clockwise on the lid 5 and around the holding means 16. By screwing, the inner walls of the clamp ring 4 come into contact with the outer walls of the holding means 16 thereby reducing a diameter of a space available at the center of the holding means 16 and further resulting in clamping the stem 28 between the holding means 16 to firmly retain the menstrual cup as can be seen in Fig. 9.

The lid 5 has a protruding portion 20 on the side opposite to the side with the holding means 16. Said protruding portion 20 enables the lid 5 to be retained in the second mounting position. On the circumference of the protruding portion 20 bumps and recesses 22 are provided that serve a function of releasing the air from the vessel 1 and ensure against an air tight seal which could result in ejecting the lid 5 if the user unintentionally pressed on the vessel 1. Once the container is opened and the lid 5 is reversed (see Fig. 10), the lid 5 is plugged by pressure and rotation in the second mounting position so as to manipulate the container with the menstrual cup attached it with ease (see Figure 9). The bumps 22 create a pressure on the flexible vessel 1 which fastens the lid 5. As can be seen in Fig. 7 a visible marker 23 saying "Do not use for drinking" indicates that the container should not be used as a drinking vessel due to bacteriological contamination. This prevents any misuse by accidental bystanders who may have found the container.

As can be seen in Fig. 3, a visible marker 19 is provided on the outside of the central ring 3 to indicate the appropriate direction of rotation to open the container.

The upper part of the vessel 1 is topped with the rim 15 which connects the vessel 1 to the central ring 3. The circular rim 15 also serves to firmly maintain the vessel 1 and the central ring 3 in place and to create a waterproof seal fully completed by an additional bulge on the rim 15 in order to apply pressure to the rim 15 and seal it when closing the container with the lid 5. Moreover, it creates a gradual pressure and gives the feeling of locking. The lid 5 and the central ring 3 are connected together by screwing in order to prevent against leakage.

The vessel 1 has the discharging means allowing the cleaning liquid and menses to be expelled after cleaning process. The discharging means comprises a bottleneck 9 arranged on the outside of the vessel 1, a spout 10, and a cap 2 with a rod 12 that enters the spout 10 and closes it tightly. The bottleneck 9 creates a waterproof seal with the cap 2. The spout 10 permits to drain off the water and thick organic materials after cleaning. The cap 2 is provided for the purpose of closing the flexible vessel at the bottleneck 9 thanks to the rod 12 entering the spout 10. A circular part located at the base of said rod 12 and a dedicated groove 11 in the bottleneck 9 help to lock them together. Moreover, the angles of the extremity of the rod 12 and narrower inner walls of the bottleneck (see detail section A-A, Fig. 8) allow to maintain the rod 12 inside once it has passed said narrower walls so it can be pulled outwards to release the cap 2 and thus free the spout 10. The flexibility of the vessel 1 allows the cap to enter the bottleneck 9 with small pressure. Additional securing means may be provided to prevent the cap 2 from unwanted and accidental unlocking. For this purpose, close to the bottleneck 9, a protruding stripe 14 with a pinch 24 is provided. The pinch 24 enters an opening 13 in a portion of the cap 2 and presses the cap 2 against the vessel 1 thereby securing the cap 1 fitted in the spout 10.

The brush 26 is arranged in the inner cavity 25 of the vessel 1 at the extremity of the bottom 8. The brush 26 extends into the bell-shaped portion 27 and is in contact or at close proximity with said portion when the lid 5 is in the first mounting position with the menstrual cup attached to it.

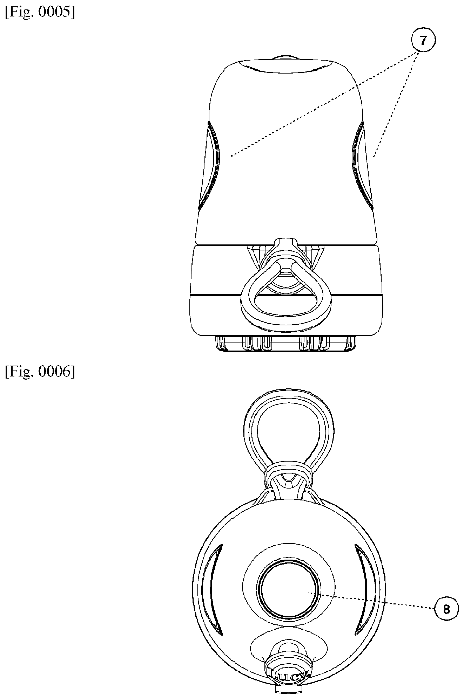

The vessel 1 may comprise visible and/or tactile markers, for instance two circular markers 7 as shown in Fig. 5 indicating where to squeeze the vessel 1 using fingers in order to clean the menstrual cup with the help of the brush 26. When squeezed, the brush 26 comes into contact with inner walls of the bell-shaped portion 27 and cleans the menstrual cup. An additional marker may be provided on the outside of the bottom 8 of the vessel 1 indicating where to press with a finger so as the brush 26 can enter into contact with the bell-shaped portion 27 in order to clean it.

The flexible string 6 is connected to the central ring 3 through an opening formed in the central ring 3, on the opposite side of the bottleneck 9 with the cap 2. The opening has a slot shape although other shapes are also possible. Said flexible string 6 enables users to hold and manipulate the container securely upon the process of cleaning and sterilization.

Although the embodiment of the present invention illustrated in the drawings comprises additional elements like a central ring, a clamp ring, a brush, etc., it would be apparent for the person skilled in the art that these elements are not necessary for the invention to solve the objective technical problem and shall not limit the scope of protection given in the appended claims.

During the process of cleaning, the container contains the menstrual cup attached to the lid 5 along with the cleaning liquid, for instance water. The menstrual cup is cleaned by squeezing, hand-shaking or pressing the markers 7 by fingers.

Sterilization must happen in a microwave oven. The level of water required for smooth and effective cleaning is indicated by the extremity of the cleaning brush 26 which normally does not exceed 60 mls. For the sterilization, the level of water can be higher in order to fully submerge the menstrual cup.

For cleaning, a quantity of water is introduced in the cavity 25 of the vessel 1 up to the level of the brush 26. Then, the lid is 5 plugged upside-down on the central ring 3 that is in the second mounting position (see Figure 10). Afterwards, the emptied menstrual cup is attached to the holding means 16 by pinching the stem 28 between the projections. Then, the user tightens the stem 28 by rotating clockwise the clamp ring 4 until the menstrual cup is firmly held. By screwing the clamp ring 4 causes the projections to bend inwards and to press against the stem 28. The lid 5 is subsequently reversed and fitted in the first mounting position with the menstrual cup inserted head down inside the vessel 1 and submerged in the water. The lid 5 is screwed on the central ring 3 to create a waterproof sealing. During the manipulation, the user operates the flexible vessel 1 by squeezing on the circular markers 7, using the thumb to press the bottom 8 to clean the inner part of the bell-shaper portion 27 with the brush 26 or by simply shaking the container.

After completing the procedure of cleaning, the cap 2 closing the spout 10 must be lifted and pulled outward thanks to the recess 21 (see Fig. 5) formed in the bottleneck 9 allowing the user to unlock the cap 2 with a finger or a nail. Beforehand, the container should be held with the cap 2 upward to avoid any used water to leak through the opened spout 10. The cleaning liquid containing menses must be expelled through the spout 10. Pressure can be applied on the flexible vessel 1 to speed up the expulsion of content and any remaining thick organic materials. When the container is empty, the cap 2 can be fitted again in the spout 10 and the lid 5 put upside down in the second mounting position. The user can safely dry the menstrual cup without having to hold it by hand (see Fig. 10).

For sterilization, the process remains the same and comprises steps of filling up the vessel 1 with water, connecting the menstrual cup to the lid 5, closing the container with the lid 5 being in the first mounting position, placing the container into the microwave oven. The cap 2 must be pulled out so as steam or boiling water can expel trough the spout 10. During the microwave process, steam produced by evaporation of the water within the container passes through the spout 10 and over the menstrual cup. On completion of the microwave cycle, residue water present in the container can simply be removed by inverting or partially inverting the container to allow drops to flow out. In addition, a dry and clean cloth can be used to absorb remaining liquid or organic materials. It is also advised to fully open and unscrew the clamp ring 4 to clean and dry the void space in between the holding means 16 and the clamp ring 4.

## Claims

1. A container for cleaning and sterilizing a menstrual cup, comprising: a vessel (1) with an open-top having a bottom (8), side walls defining an inner cavity (25) for holding a cleaning liquid and for receiving a menstrual cup having gripping means (28);
a lid (5) closing the open-top;
the container being made of materials suitable for exposure to microwave radiation, **characterized in that** the lid (5) comprises holding means (16) for connecting the menstrual cup to the lid (5) with the gripping means (28), wherein the lid (5) can be fitted on the open-top in a first mounting position with the holding means (16) directed towards the inner cavity (25) such that, when attached to the lid (5), the menstrual cup projects at least partially into the inner cavity (25), and
wherein the lid (5) can be fitted in a second mounting position with the lid (5) reversed and the holding means (16) directed away from the inner cavity (25) such that, when attached to the lid (5), the menstrual cup projects outwardly.

2. A container according to claim 1, wherein the container is made of elastic material.

3. A container according to claim 2, wherein the elastic material is silicone or polymer or co-polymer plastic materials or combination thereof.

4. A container according to any preceding claims 1 to 3, wherein the holding means (16) is in the form of projections suitable for pinching the gripping means (28) and retaining the menstrual cup firmly in place.

5. A container according to any preceding claims 1 to 4, wherein a central ring (3) is provided between the vessel (1) and the lid (2), the central ring (3) encloses an upper part of the vessel (1) and together with the lid (5) forms a tight connection.

6. A container according to any preceding claims 1 to 5, wherein a clamp ring (4) with a portion at least partially enclosing the holding means (16) is attached to the lid (5) by screwing such that as the clamp ring (4) screws on the lid (5) said portion tightens up the gripping means (28) and retains the menstrual cup firmly in place.

7. A container according to any preceding claims 1 to 6, comprises a brush (26) arranged in the inner cavity (25) of the vessel (1), wherein the brush (26) extends into a bell-shaped portion (27) of the menstrual cup and is in contact or at close proximity with said portion when the lid (5) is in the first mounting position with the menstrual cup attached to it.

8. A container according to any preceding claims 1 to 7, wherein the vessel (1) is provided with discharging means allowing the cleaning liquid and menses to be expelled after cleaning process and allowing a steam to be expelled during sterilization.

9. A container according to claim 8, wherein the discharging means comprises a bottleneck (9) arranged on the outside of the vessel (1), a spout (10), and a cap (2) with a rod (12) that enters the spout (10) and closes it tightly.

10. A container according to claim 9, wherein the bottleneck (9) is provided with a recess (21) that serves to pry and unlock the cap (2).

11. A container according to claim 9 and 10, wherein on the outside of the vessel (1), close to the bottleneck (9), a protruding stripe (14) with a pinch (24) is provided, the pinch (24) enters an opening (13) in a portion of the cap (2) and presses the cap (2) against the vessel (1) thereby securing the cap (1) fitted in the spout (10).

12. A container according to any preceding claims 1 to 11, wherein the lid (5) has a protruding portion (20) on the side opposite to the side with the holding means (16), the protruding portion (20) enables the lid (5) to be retained in the second mounting position.

13. A container according to claim 12 wherein the protruding portion (20) has on its circumference bumps and recesses (22) that serve a function of releasing the air from the vessel (1) and ensure against an air tight seal.

14. A container according to any preceding claims 1 to 13, comprises a flexible string (6) that provides for secure and easy manipulating with user hands.

## Patentansprüche

1. Behälter zum Reinigen und Sterilisieren einer Menstruationstasse, welcher Folgendes umfasst: ein Gefäß (1) mit einer offenen Oberseite mit einem Boden (8), Seitenwänden, die einen inneren Hohlraum (25) zur Aufnahme einer Reinigungsflüssigkeit und zur Aufnahme einer Menstruationstasse mit Greifmitteln (28) definieren;
einen Deckel (5), der die offene Oberseite verschließt;
wobei der Behälter aus Materialien hergestellt ist, die geeignet sind, Mikrowellenstrahlung ausgesetzt zu werden, **dadurch gekennzeichnet, dass** der Deckel (5) Haltemittel (16) zum Verbinden der Menstruationstasse mit dem Deckel (5) mit den Greifmitteln (28) umfasst, wobei der Deckel (5) auf der offenen Oberseite in einer ersten Montageposition angebracht werden kann, wobei die Haltemittel (16) zu dem inneren Hohlraum (25) gerichtet sind, so dass die Menstruationstasse, wenn sie an dem Deckel (5) angebracht ist, zumindest teilweise in den inneren Hohlraum (25) hineinragt, und
wobei der Deckel (5) in einer zweiten Montageposition angebracht werden kann, in der der Deckel (5) umgedreht ist und das Haltemittel (16) vom inneren Hohlraum (25) weggerichtet ist, so dass die Menstruationstasse, wenn sie am Deckel (5) angebracht ist, nach außen ragt.

2. Ein Behälter nach Anspruch 1, wobei der Behälter aus elastischem Material hergestellt ist.

3. Ein Behälter nach Anspruch 2, wobei das elastische Material Silikon oder Polymer oder Co-Polymer-Kunststoffmaterialien oder eine Kombination davon ist.

4. Ein Behälter nach einem der vorhergehenden Ansprüche 1 bis 3, wobei das Haltemittel (16) die Form von Vorsprüngen hat, die geeignet sind, das Greifmittel (28) einzuklemmen und die Menstruationstasse fest in ihrer Position zu halten.

5. Ein Behälter nach einem der vorhergehenden Ansprüche 1 bis 4, wobei zwischen dem Gefäß (1) und dem Deckel (2) ein Mittelring (3) vorgesehen ist, wobei der Mittelring (3) einen oberen Teil des Gefäßes (1) umschließt und zusammen mit dem Deckel (5) eine dichte Verbindung bildet.

6. Ein Behälter nach einem der vorhergehenden Ansprüche 1 bis 5, **dadurch gekennzeichnet, dass** ein Klemmring (4) mit einem das Haltemittel (16) zumindest teilweise umschließenden Abschnitt durch Verschraubung mit dem Deckel (5) derart verbunden ist, dass beim Aufschrauben des Klemmrings (4) auf den Deckel (5) dieser Abschnitt das Greifmittel (28) festzieht und die Menstruationstasse fest in ihrer Position hält.

7. Ein Behälter nach einem der vorhergehenden Ansprüche 1 bis 6, umfassend eine Bürste (26), die im inneren Hohlraum (25) des Gefäßes (1) angeordnet ist, wobei die Bürste (26) in einen glockenförmigen Abschnitt (27) der Menstruationstasse hineinragt und mit diesem Abschnitt in Kontakt oder in unmittelbarer Nähe ist, wenn sich der Deckel (5) in der ersten Montageposition befindet und die Menstruationstasse daran befestigt ist.

8. Ein Behälter nach einem der vorhergehenden Ansprüche 1 bis 7, wobei das Gefäß (1) mit einem Ablassmittel versehen ist, die es ermöglicht, die Reinigungsflüssigkeit und die Menstruation nach dem Reinigungsvorgang auszustoßen und während der Sterilisation einen Dampf auszustoßen.

9. Ein Behälter nach Anspruch 8, wobei das Ablassmittel einen an der Außenseite des Behälters (1) angeordneten Flaschenhals (9), eine Tülle (10) und eine Kappe (2) mit einer Stange (12) umfasst, die in die Tülle (10) eintritt und diese dicht verschließt.

10. Ein Behälter nach Anspruch 9, **dadurch gekennzeichnet, dass** der Flaschenhals (9) mit einer Ausnehmung (21) versehen ist, die zum Aufhebeln und Entriegeln der Kappe (2) dient.

11. Ein Behälter nach Anspruch 9 und 10, wobei an der Außenseite des Gefäßes (1) in der Nähe des Flaschenhalses (9) ein vorstehender Streifen (14) mit einer Quetschung (24) vorgesehen ist, wobei die Quetschung (24) in eine Öffnung (13) in einem Teil der Kappe (2) eindringt und die Kappe (2) gegen das Gefäß (1) drückt, wodurch die in die Tülle (10) eingesetzte Kappe (1) gesichert wird.

12. Ein Behälter nach einem der vorhergehenden Ansprüche 1 bis 11, wobei der Deckel (5) auf der Seite, die der Seite mit dem Haltemittel (16) gegenüberliegt, einen vorstehenden Abschnitt (20) aufweist, wobei der vorstehende Abschnitt (20) es ermöglicht, den Deckel (5) in der zweiten Montageposition zu halten.

13. Ein Behälter nach Anspruch 12, wobei der vorstehende Abschnitt (20) an seinem Umfang Erhebungen und Vertiefungen (22) aufweist, die dazu dienen, die Luft aus dem Gefäß (1) abzulassen und einen luftdichten Verschluss zu gewährleisten.

14. Ein Behälter nach einem der vorhergehenden Ansprüche 1 bis 13, umfassend eine flexible Schnur (6), die für eine sichere und einfache Handhabung mit den Händen des Benutzers sorgt.

## Revendications

1. Un récipient pour nettoyer et stériliser une coupe menstruelle, comprenant : un vaisseau (1) avec un dessus ouvert ayant un fond (8), des parois latérales définissant une cavité intérieure (25) pour contenir un liquide de nettoyage et pour recevoir une coupe menstruelle ayant des moyens de préhension (28); un couvercle (5) fermant le dessus ouvert ;
le récipient étant constitué de matériaux adaptés à l'exposition au rayonnement micro-ondes, **caractérisé en ce que** le couvercle (5) comprend des moyens de maintien (16) pour relier la coupe menstruelle au couvercle (5) à l'aide des moyens de préhension (28), où le couvercle (5) peut être monté sur le dessus ouvert dans une première position de montage avec les moyens de maintien (16) orientés vers la cavité intérieure (25) de telle sorte que, lorsqu'elle est fixée au couvercle (5), la coupe menstruelle fait saillie au moins partiellement dans la cavité intérieure (25), et
où le couvercle (5) peut être monté dans une seconde position de montage avec le couvercle (5) inversé et les moyens de maintien (16) orientés à l'opposé de la cavité intérieure (25) de telle sorte que, lorsqu'elle est fixée au couvercle (5), la coupe menstruelle fait saillie vers l'extérieur.

2. Un récipient selon la revendication 1, dans lequel le récipient est constitué d'un matériau élastique.

3. Le récipient selon la revendication 2, dans lequel le matériau élastique est du silicone ou des matières plastiques polymères ou copolymères ou une combinaison de ceux-ci.

4. Un récipient selon l'une des revendications précédentes 1 à 3, dans lequel les moyens de maintien (16) se présentent sous la forme de saillies adaptées pour pincer les moyens de préhension (28) et de maintenir la coupe menstruelle fermement en place.

5. Un récipient selon l'une quelconque des revendications précédentes 1 à 4, dans lequel un anneau central (3) est prévu entre le vaisseau (1) et le couvercle (2), l'anneau central (3) entoure une partie supérieure du vaisseau (1) et, avec le couvercle (5), forme une connexion étanche.

6. Un récipient selon l'une quelconque des revendications précédentes 1 à 5, dans lequel un anneau de serrage (4) dont une partie renferme au moins partiellement les moyens de maintien (16) est fixé au couvercle (5) par vissage de telle sorte que lorsque l'anneau de serrage (4) est vissé sur le couvercle (5), cette partie resserre les moyens de préhension (28) et maintient la coupe menstruelle fermement en place.

7. Un récipient selon l'une quelconque des revendications précédentes 1 à 6, comprend une brosse (26) disposée dans la cavité intérieure (25) du vaisseau (1), où la brosse (26) s'étend dans une partie en forme de cloche (27) de la coupe menstruelle et est en contact ou à proximité de ladite partie lorsque le couvercle (5) est dans la première position de montage avec la coupe menstruelle fixée à celui-ci.

8. Un récipient selon l'une quelconque des revendications précédentes 1 à 7, dans lequel le vaisseau (1) est pourvu de moyens de décharge permettant au liquide de nettoyage et aux menstrues d'être expulsées après le processus de nettoyage et permettant à la vapeur d'être expulsée pendant la stérilisation.

9. Un récipient selon la revendication 8, dans lequel les moyens de décharge comprennent un goulot d'étranglement (9) disposé à l'extérieur du récipient (1), un bec (10) et un bouchon (2) avec une tige (12) qui pénètre dans le bec (10) et le ferme étanchement.

10. Un récipient selon la revendication 9, dans lequel le goulot d'étranglement (9) est pourvu d'un évidement (21) qui sert à soulever et à déverrouiller le bouchon (2).

11. Un récipient selon les revendications 9 et 10, dans lequel sur l'extérieur du récipient (1), près du goulot d'étranglement (9), une bande saillante (14) avec une pince (24) est prévue, la pince (24) pénètre dans une ouverture (13) dans une partie du bouchon (2) et presse le bouchon (2) contre le récipient (1), fixant ainsi le bouchon (1) installé dans le bec (10).

12. Un récipient selon l'une des revendications précédentes 1 à 11, dans lequel le couvercle (5) a une partie saillante (20) sur le côté opposé au côté avec les moyens de maintien (16), la partie saillante (20) permet au couvercle (5) d'être maintenu dans la seconde position de montage.

13. Un récipient selon la revendication 12, dans lequel la partie saillante (20) a sur sa circonférence des bosses et des évidements (22) qui ont pour fonction de dégager l'air du récipient (1) et d'assurer une étanchéité à l'air.

14. Un récipient selon l'une quelconque des revendications précédentes 1 à 13, comprend une ficelle flexible (6) qui permet une manipulation sûre et facile avec les mains de l'utilisateur.
